# EUROPEAN PATENT APPLICATION

(11) **EP 2 003 142 A1**
(43) Date of publication of application: **17.12.2008**
(21) Application number: 08162970.1
(22) Date of filing: 13.01.2005
(51) Int. Cl.: C07K 5/062, C07D 209/42, A61K 38/05, A61P 9/00

(54) **Amorphous perindopril erbumine**

(30) Priority: 14.01.2004 SI 200400011
(62) Divisional of application: 05700892.2
(71) Applicant: Lek Pharmaceuticals D.D., 1526 Ljubljana (SI)
(72) Inventor: Rucman, Rudolf, 1211, Ljubljana-Smartno (SI)
(74) Representative: Hodzar, Damjan

(57) **Abstract**

Amorphous perindopril erbumine is claimed.

## Description

### Field of the invention

The present invention relates to new complexes of the ACE inhibitor perindopril, a salt, an addition salt or a derivative thereof with polymers, and to a process for the preparation of such complexes. Further, the invention relates to amorphous perindopril erbumine.

### Background of the Invention

Perindopril is a dipeptide compound, composed of two amino acids with 5 chiral centres in the molecule:

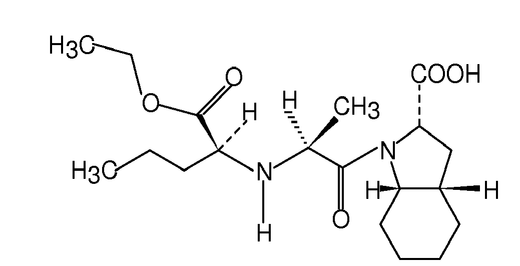

Perindopril was first described in EP 0049658 B1 and US 4,508,729 as optically pure S,S isomer and in the form of sodium salt.
Processes for the preparation of perindopril are disclosed by numerous patents and patent applications, such as: EP 0308 341 B1, EP 1 279 665 A1, SI 9 500 140, and others.

EP 1296947 B1, EP 1294689 A and EP 1296948 B1 disclose α, β and γ crystalline forms of perindopril, respectively.

Perindopril is chemically highly sensitive and may isomerise in protic solvents at some chiral centres resulting in formation of less active compounds. Various substituted diketopiperazides may be formed by oxidation and inner cyclization.

Moreover, the peptide, as well as the ester bond, are sensitive to hydrolysis. For this reason it is desirable to develop new ways of stabilizing perindopril.

### Summary of the invention

In one embodiment the present invention relates to a complex of perindopril, a salt, an addition salt or derivative thereof with a polymer, wherein said complex is selected from the group consisting of inclusion complexes with cyclodextrins or their alkylated and hydroxyalkylated derivatives, and complexes with water-soluble polymers selected from the group consisting of polyvinylpyrrolidone and hydroxyalkylcellulose derivatives.

In another embodiment of the present invention amorphous perindopril erbumine is provided.

In another embodiment the present invention relates to a process for the preparation of a complex of perindopril, a salt, an addition salt or a derivative thereof with polymer comprising:
- (i) dissolving a polymer in water or a water/alcoholic solution,
- (ii) adding perindopril, a salt, an addition salt or a derivative thereof to the solution,
- (iii) stirring the mixture at a temperature in the range from about 20°C to 60°C,
- (iv) filtering, and
- (v) drying.

In another embodiment the present invention relates to a pharmaceutical composition comprising a therapeutically effective amount of the complex of perindopril or a salt, an addition salt or a derivative thereof with a polymer.

In another embodiment the present invention relates to use of a complex of perindopril, or a salt, an addition salt or a derivative thereof with a polymer for the preparation of a pharmaceutical composition for use in the treatment of cardiovascular diseases.

### Description of the Figures

Figure 1. NMR spectrum of perindopril erbumine inclusion complex with β-cyclodextrin.
Figure 2. NMR spectrum of perindopril erbumine.
Figure 3. NMR spectrum of β-cyclodextrin.
Figure 4. NMR spectrum of perindopril erbumine inclusion complex with methyl-β-cyclodextrin.
Figure 5. NMR spectrum of methyl-β-cyclodextrin.
Figure 6. NMR spectrum of perindopril erbumine inclusion complex with ε-cyclodextrin.
Figure 7. NMR spectrum of perindopril erbumine inclusion complex with polyvinylpyrrolidone.
Figure 8. DSC thermogram of physical mixture of perindopril erbumine and β-cyclodextrin.
Figure 9. DSC thermogram of perindopril erbumine inclusion complex with β-cyclodextrin.
Figure 10. DSC thermogram of perindopril erbumine inclusion complex with methyl-β-cyclodextrin.
Figure 11. DSC thermogram of perindopril erbumine inclusion complex with hydroxypropyl-β-cyclodextrin.
Figure 12. DSC thermogram of perindopril erbumine complex with hydroxypropylcellulose.
Figure 13. DSC thermogram of perindopril erbumine inclusion complex with γ-cyclodextrin.
Figure 14. X-ray diffraction diagram of pure β-cyclodextrin.
Figure 15. X-ray diffraction diagram of inclusion complex of perindopril erbumine with β-cyclodextrin.
Figure 16. X-ray diffraction diagram of pure methyl-β-cyclodextrin.
Figure 17. X-ray diffraction diagram of perindopril erbumine inclusion complex with methyl-β-cyclodextrin.
Figure 18. X-ray diffraction diagram of perindopril erbumine inclusion complex with hydroxypropyl -β-cyclodextrin.
Figure 19. X-ray diffraction diagram of perindopril erbumine inclusion complex with γ-cyclodextrin.
Figure 20. IR spectrum of inclusion complex of perindopril erbumine with hydroxypropyl -β-cyclodextrin.
Figure 21. IR spectrum of inclusion complex of perindopril erbumine with hydroxypropylcellulose.
Figure 22. IR spectrum of inclusion complex of perindopril erbumine with γ-cyclodextrin.
Figure 23. IR spectrum of inclusion complex of perindopril erbumine with polyvinylpyrrolidone.

### Detailed description of the invention

One aspect of the present invention concerns novel inclusion complexes of perindopril, a salt, an addition salt or a derivative thereof with cyclodextrins. The preferred addition salt of perindopril for use in the invention is perindopril erbumine.

We have surprisingly found that the stability of perindopril, a salt, an addition salt or a derivative thereof is increased by including them in the inner cavity of the cyclodextrin cyclic structure, where they are protected from external influences.

Among various cyclodextrins α-, β-, γ- and ε-cyclodextrins or their methylated or hydroxyalkylated derivatives may be used. Methods for preparation of the inclusion complexes are described below. Similar preparative process can be used irrespective of whether α-, β-, γ- and ε-cyclodextrins, or their methylated or hydroxyalkylated derivates are used.

In another aspect we have found that besides cyclodextrins also other polymeric compounds may be used in the formation of complexes with perindopril, a salt, an addition salt or a derivative thereof. Preferably polymers are selected from the group consisting of polyvinylpyrrolidone (PVP), especially PVP with molecular weight between 10,000 and 40,000, and hydroxyalkyl cellulose derivatives, preferably hydroxypropylcellulose (HPC). Perindopril, a salt, an addition salt or a derivative thereof, which are included inside the three-dimensional net of molecular fibres are stabilized and protected from external influences.

A "complex" in the present invention means that perindopril, a salt, an addition salt or a derivative thereof is included inside the three-dimensional net of polymer fibres.

An "inclusion complex" in the present invention means that perindopril, a salt, an addition salt or a derivative thereof is included in the inner cavity of the cyclodextrin cyclic structure.

In another embodiment the present invention relates to a process for the preparation of a complex of perindopril, a salt, an addition salt or a derivative thereof with polymer comprising:
- (i) dissolving a polymer in water or a water/alcoholic solution,
- (ii) adding perindopril, a salt, an addition salt or a derivative thereof to the solution,
- (iii) stirring the mixture at a temperature in the range from about 20°C to 60°C,
- (iv) filtering, and
- (v) drying.

The first step of one process suitable for the preparation of the complex according to the invention involves mixing polymer with water or a water/alcohol mixture and then adding perindopril, a salt, an addition salt or a derivative thereof. The solution is then stirred for a period of time e.g. from 1 to 3 hours, for instance at a temperature in the range from 20°C to 60°C, e.g. 20°C to 30°C, until the perindopril, a salt, an addition salt or a derivative thereof are inserted into the complex. The solution is then filtered and dried afterwards in an appropriate manner. Preferably, lyophilization or spray-drying are suitable drying methods.
The ratio between perindopril and polymer is not critical and usually depends on the desired final dose of perindopril in tablets or capsules. Optionally the complexes comprise from 10 % to 50 % by weight of perindopril according to the total weight of the complex. Inclusion complexes with cyclodextrins preferably comprise from 15 % to 25 % by weight of perindopril according to the total weight of the complex, more preferably from 18 % to 22 % by weight of perindopril according to the total weight of the complex. Complexes with PVP preferably comprise from 11 % to 22 % by weight of perindopril according to the total weight of the complex, more preferably from 15 % to 18 % by weight of perindopril according to the total weight of the complex. Complexes with HPC preferably comprise from 15 % to 25 % by weight of perindopril according to the total weight of the complex, more preferably from 18 % to 22 % by weight of perindopril according to the total weight of the complex.

The complexes can be characterized by IR and NMR spectra, DSC thermograms, and X-ray powder diffraction diagrams.

The content of perindopril in the complexes can be determined by suitable HPLC analysis or by modified signals in IR and NMR spectra.

The determination of the melting point of inclusion complexes shows that, in fact, they are not simple mechanical mixtures of both components. In particular, no melting point characteristic of perindopril is observed. The melting point is, considerably higher than that of perindopril, but typically lower than that of pure carrier, e.g. cyclodextrin.

For instance, in the case of the inclusion complex of perindopril erbumine with methyl-#-cydodextrin the melting points (determined with the Koffler microscope) are as follows:
methyl-β-cyclodextrin 186 - 192° C
perindopril erbumine 118-130° C
inclusion complex 148 -156° C.

Transition states from solid to liquid phase of amorphous substances are in fact glass transition states - not melting points - although there is no visual difference during Koffler microscopy. Therefore, while melting temperatures of methyl-β-cyclodextrin and perindopril erbumine are melting points, the melting temperature of the inclusion complex is a glass transition temperature. All melting temperatures mentioned herein must be interpreted with this in mind.

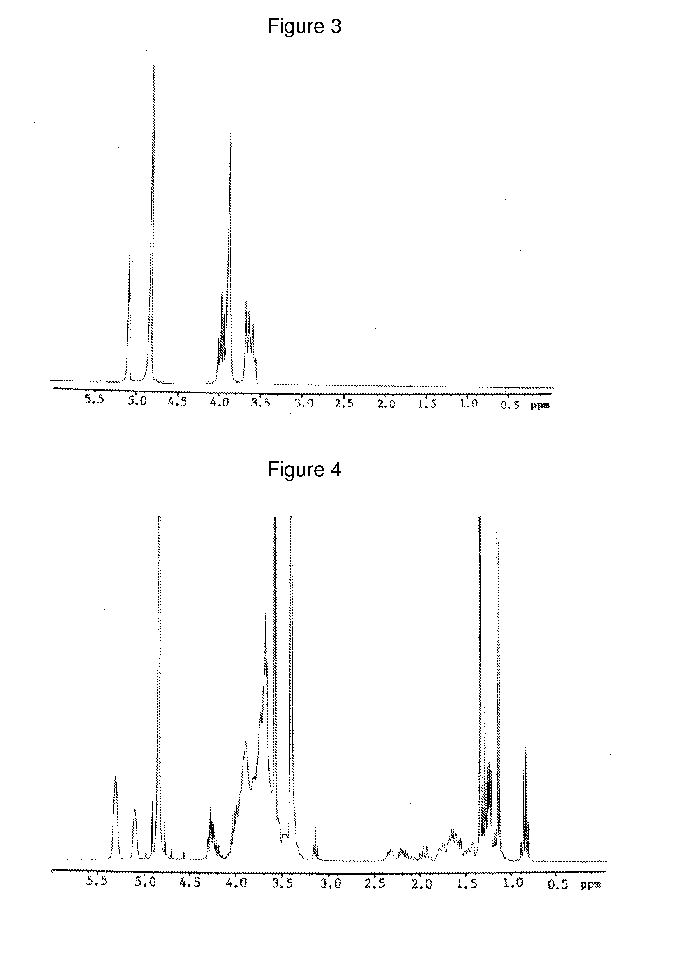

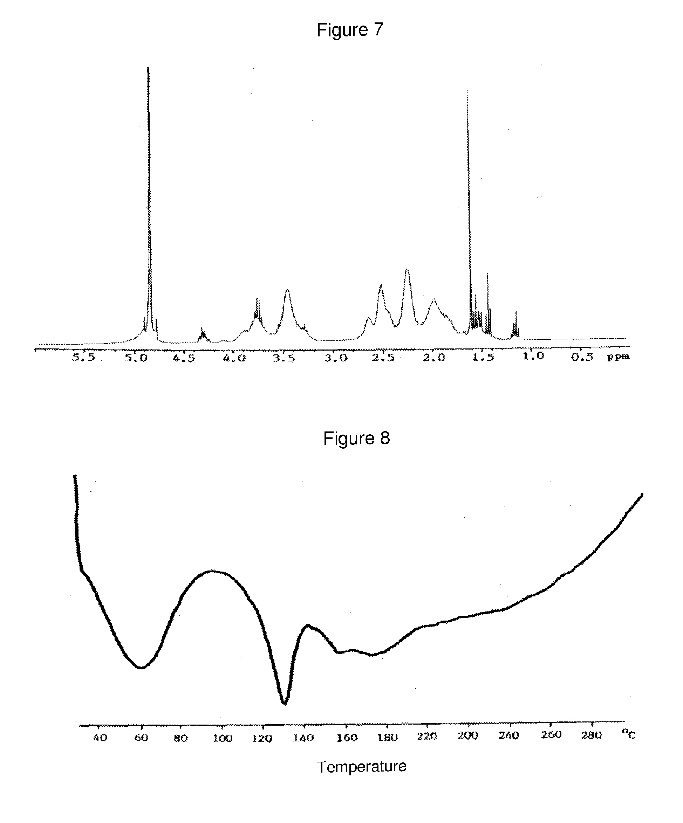

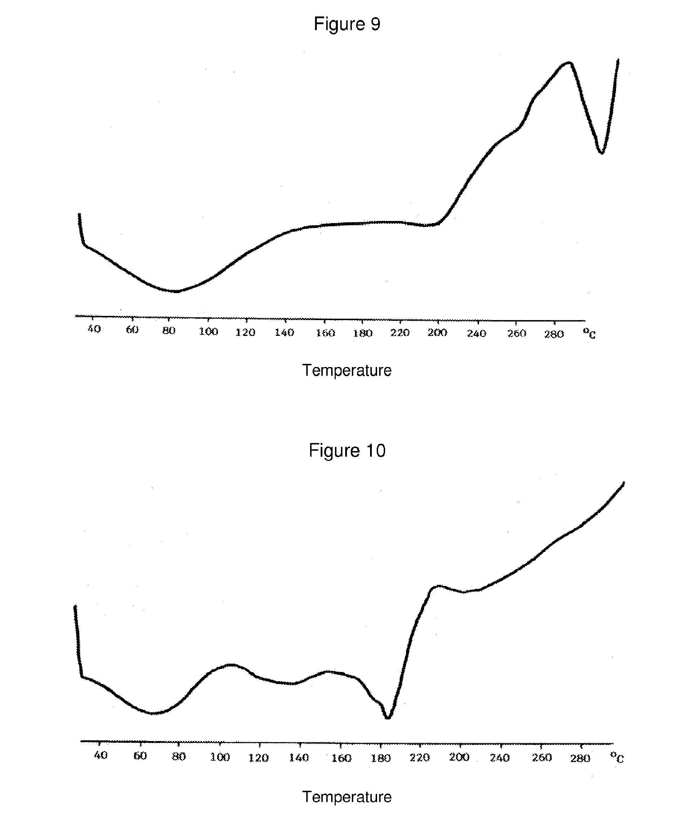

The thermic process in heating the inclusion complexes is also illustrated by DSC thermograms (Figures 9-13), in which no endothermic transitions characteristic for a purely physical mixture of both components (Figure 8) can be detected.
The NMR spectrum of perindopril erbumine inclusion complex shows the signals characteristic for perindopril (at 0.88; 1,33 and 3.1 ppm) and the signals characteristic of cyclodextrin (in the case of methyl-β-cyclodextrin at 3.38 ppm, singlet; 3.54 ppm, singlet; 5.55 ppm, broad singlet and 5.28 ppm - broad singlet) (Figure 4).

The powder X-ray diffraction pattern of the perindopril erbumine inclusion complex shows no characteristic peaks, thereby identifying that the amorphous structure is present. Perindopril erbumine and cyclodextrin are therefore not present as a mere physical mixture of two different crystals but in the form of a new homogenous amorphous material stabilized by incorporation of the perindopril moiety inside a cyclodextrin cylinder. In polymeric materials such as polyvinylpirrolidone and hydroxypropylcellulose perindopril erbumine is caught inside the net of molecular fibres what again stabilizes the amorphous form.

In another embodiment of the present invention amorphous perindopril erbumine is provided. Amorphous perindopril erbumine is stabilized by incorporation into a complex according to the present invention.

In one embodiment the present invention relates to a complex of perindopril, a salt, an addition salt or derivative thereof with a polymer, wherein said complexes are selected from the group consisting of inclusion complexes with cyclodextrins or their alkylated and hydroxyalkylated derivatives, and complexes with water-soluble polymers selected from the group consisting of polyvinylpyrrolidone and hydroxyalkylcellulose derivatives, obtainable according to a process of the present invention.

In one embodiment the present invention relates to a complex of perindopril, a salt, an addition salt or derivative thereof with a polymer, wherein said complexes are selected from the group consisting of inclusion complexes with cyclodextrins or their alkylated and hydroxyalkylated derivatives, and complexes with water-soluble polymers selected from the group consisting of polyvinylpyrrolidone and hydroxyalkylcellulose derivatives, obtained by a process of the present invention.

Perindopril erbumine, which is an addition salt of perindopril with tert-butylamine, is chemically 2-methylpropane-2-ammonium (2S,3aS,7aS)-1-(((1S)-1-(ethoxycarbonyl)butyl)amino)propionyl)octahydro-1H-indole-2-carboxylate. As an effective ACE (Angiotensin Converting Enzyme) inhibitor it is used in the treatment of cardiovascular diseases, e.g. hypertension or heart failure. It acts as a transport form of active perindoprilat, which is formed after splitting the ester bond.

In another embodiment the present invention also relates to pharmaceutical compositions comprising a therapeutically effective amount of a complex of perindopril, a salt, an addition salt or a derivative thereof with a polymer, or amorphous perindopril erbumine, together with one or more pharmaceutically acceptable carriers or other excipients.

A therapeutically effective amount of such a complex is the amount of complex which comprises an amount of perindopril which is appropriate in a dosage form useful to treat hypertension or cardiovascular diseases. In general, from a pharmaceutically effective amount is 1 to 15 mg of perindopril, preferably 2 to 8 mg.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of hydroxypropylcellulose, lactose, microcrystalline cellulose, calcium carbonate, starch, colloidal silicone dioxide, sodium starch glycolate, talc, magnesium stearate, polyvinylpyrrolidone, and other excipients known in the field of the pharmaceutical technology.

Optionally, the pharmaceutical compositions of the invention may be combination products comprising one or more additional pharmaceutically active components in addition to perindopril. Preferably, an additional pharmaceutically active component is a diuretic, e.g. indapamide.

Suitable pharmaceutical compositions are solid dosage forms, such as tablets with immediate release or sustained release of the active principle, effervescent tablets or dispersion tablets and capsules.

The pharmaceutical compositions may be prepared by methods known in the field of the pharmaceutical technology.

In another embodiment the present invention relates to use of a complex of perindopril, a salt, an addition salt or a derivative thereof with a polymer for the preparation of a pharmaceutical composition for use in the treatment of cardiovascular diseases, e.g. hypertension or heart failure.

The following examples illustrate the invention, but do not limit it in any way:

### Examples

### Inclusion complex of perindopril erbumine with β-cyclodextrin

### Example 1

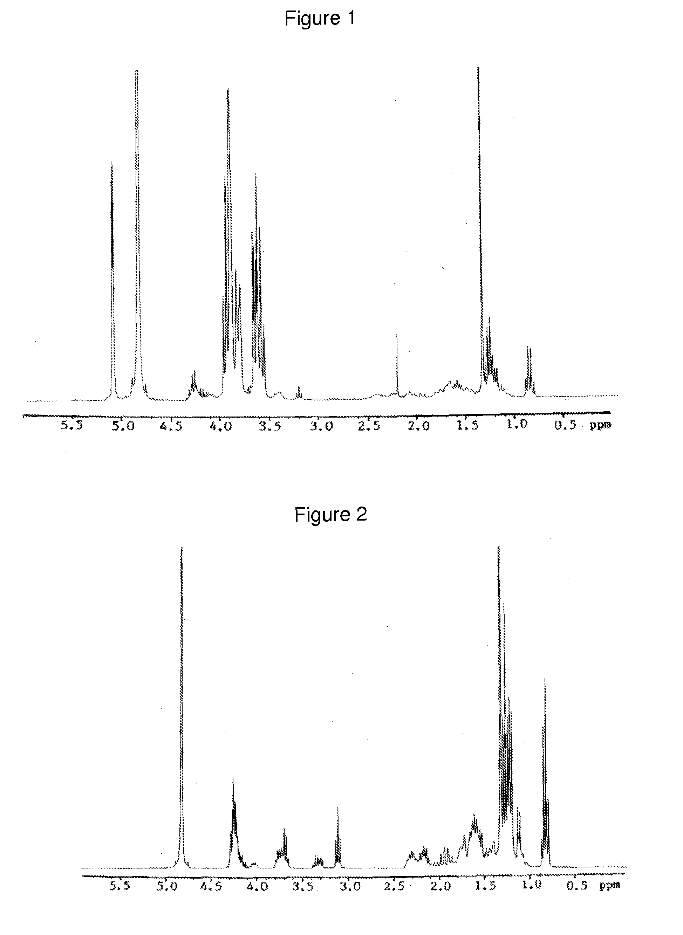

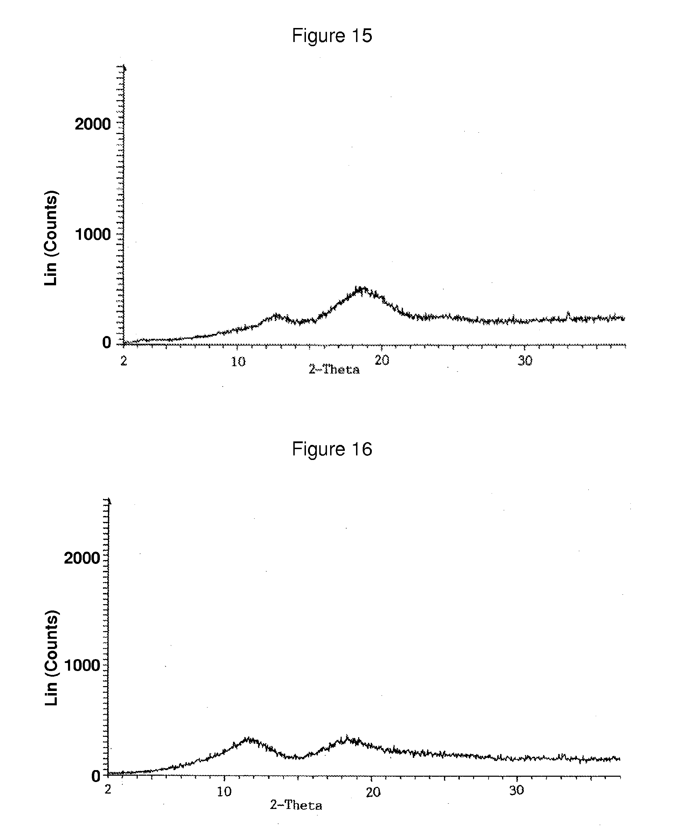

4.0 g of β-cyclodextrin are dissolved in 18 ml of water. 1.0 g of perindopril erbumine is added under stirring at 50°C until dissolved and stirring is continued for one hour at this temperature. The slightly turbid solution is filtered through a sterile 0.2 µm filter directly into a lyophilization flask, frozen and lyophilized. 4.49 g (90 % yield) of dry matter is obtained, which does not show any evidence of melting at temperatures up to 290°C, where it turns brown and disintegrates in the same way as the pure β-cyclodextrin starting material.
The NMR spectrum (D₂0) (Figure 1) shows signals characteristic for perindopril erbumine (0.88 ppm, quartet; 1.33 ppm, singlet, 3.10 ppm, triplet) and signals highly characteristic for β-cyclodextrin (multiplets at 3.6 and 3.85 ppm and doublet at 5.06 ppm). The powder X-ray diffraction diagram (Figure 15) does not show a crystalline structure, which is understandable with regard to the method of production, i.e. lyophilization.

### Example 2

4.0 g of β-cyclodextrin is putted into 20 ml of water and then while stirring 1.0 g of perindopril erbumine is added. The mixture is stirred at 50°C until most of material is dissolved. After filtration the clear solution is spray-dried at an inlet air temperature of 120°C. The amorphous product which is collected from the apparatus shows differences from a physical mixture of β-cyclodextrin and perindopril erbumine.

### Example 3

4.0 g of β-cyclodextrin are dissolved in 18 ml of water. 0.94 g of perindopril is added under stirring at 50°C until dissolved, the mixture is filtered and cooled to room temperature, then 0.06 g of tert-butylamine is added by syringe and after further stirring for 5 min, the solution is transferred into a lyophilization flask, frozen and lyophilized. 4.47 g (88 % yield) of dry matter is obtained, which does not show any melting at temperature of up to 290°C, where it turns brown and disintegrates in the same way as the pure β-cyclodextrin starting material.

### Inclusion complex of perindopril erbumine with methyl-β-cyclodextrin

### Example 4

4.0 g of methyl-β-cyclodextrin and 1.0 g of perindopril erbumine are dissolved in 50 ml of water and 6 ml of isopropanol. The mixture is stirred at room temperature for 1 hour and filtered through a sterile 0.2 µm filter, frozen and lyophilized. 4.41 g (88.2 % yield) of substance which melts at 148-156 °C is obtained.

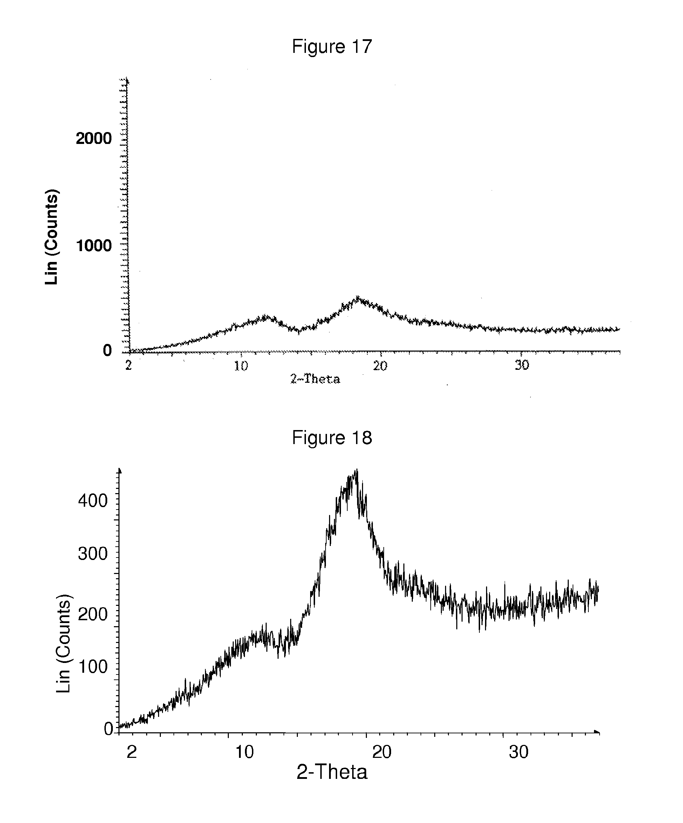

The NMR spectrum (D₂0) (Figure 4) shows signals characteristic for perindopril (at 0.88; 1.33 and 3.1 ppm) and signals characteristic of methyl-β-cyclodextrin (3.38 ppm, singlet; 3.54 ppm, singlet; 5.55 ppm, broad singlet and 5.28 ppm - broad singlet). The powder X-ray diffraction diagram (Figure 17) shows an amorphous structure.

### Inclusion complex of perindopril erbumine with hydroxypropyl-β-cyclodextrin complex

### Example 5

20.0 g of hydroxypropyl-β-cyclodextrin is stirred in 75 ml of water and then 5.0 g of perindopril erbumine is added. The mixture is stirred at room temperature until most of the material is dissolved. The clear solution is freeze-dried for 24 hours and 24.9 g of amorphous product is collected. The complex melts and disintegrates at 235-245°C.

### Example 6

12.0 g of hydroxypropyl-β-cyclodextrin is stirred into 50 ml of water and then 3.0 g of perindopril erbumine is added. The mixture is stirred at room temperature until most of the material is dissolved. After filtration the clear solution is spray-dried at an air inlet temperature of 120°C. The amorphous product which is collected from the apparatus shows differences from a physical mixture of hydroxypropyl-β-cyclodextrin and perindopril erbumine.

### Inclusion complex of perindopril erbumine with γ-cyclodextrin

### Example 7

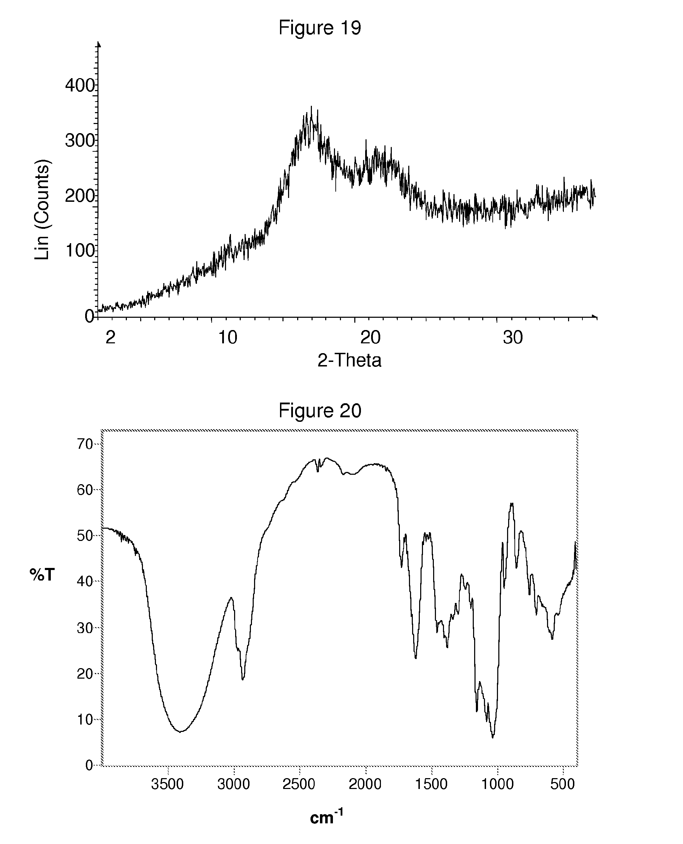

4.0 g of γ-cyclodextrin is dissolved in 20 ml of water and 1.0 g of perindopril erbumine is added. The mixture is stirred at ambient temperature until dissolved. The solution is frozen and immediately lyophilized for 24 hours. The product (4.6 g) is amorphous (Figure 19) and shows differences from a physical mixture of γ-cyclodextrin and perindopril erbumine. It has no melting phenomenon and disintegrates at about 220°C.

### Inclusion complex of perindopril erbumine with ε-cyclodextrin

### Example 8

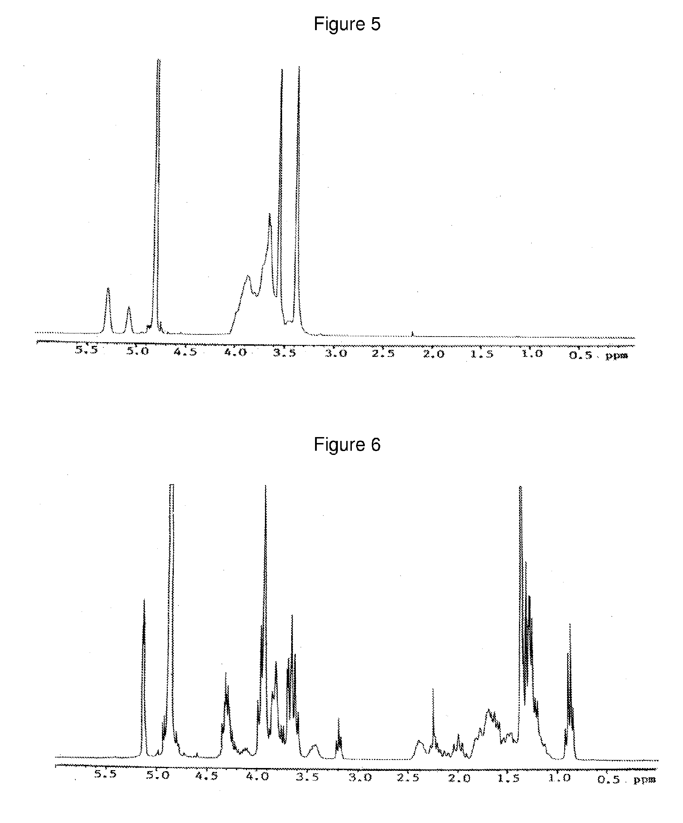

500 mg of ε-cyclodextrin and 500 mg of perindopril erbumine are dissolved in 10 ml of water. The mixture is stirred for 1 hour at 30°C, filtered, frozen and lyophilized. 0.93 g (93 %) of white substance is obtained, which has no sharp melting point, but turns brown above 250°C and disintegrates. The NMR spectrum (Figure 6) of complex shows signals characteristic of both components. The powder X-ray diffraction diagram shows amorphous substance.

### Complex of perindopril erbumine with polyvinylpyrrolidone

### Example 9

5 g of polyvinylpyrrolidone K-30 with molecular weight 40 000 and 1 g of perindopril erbumine are dissolved in 60 ml of water and 6 ml of isopropanol. The mixture is finally filtered (2 µm) and spray-dried at 120°C. The dry substance with a weight 5.9 g (98 %) melts at 170-195°C. The NMR spectrum (Figure 7) of complex shows signals characteristic of both components. The powder X-ray diffraction diagram shows amorphous substance.

### Example 10

5.0 g of polyvinylpyrrolidone with molecular weight 10.000 is stirred in 20 ml of water and 1.0 g of perindopril erbumine is added. The mixture is finally filtered (2 µm) and freeze-dried for 24 hours. The dry substance with a weight 5.7 g has a glass transition temperature at 153-170°C. This complex also shows characteristic physico-chemical characteristics signals of both components and is amorphous.

### Complex of perindopril erbumine with hydroxypropylcellulose

### Example 11

4.0 g of hydroxypropylcellulose and 1.0 g of perindopril erbumine is mixed with 150 ml of water and stirred at ambient temperature until dissolved. The solution is afterwards filtered, frozen immediately and lyophilized for 24 hours. The dry substance with a weight of 4.8 g is amorphous and has a glass transition temperature at 130-150 °C.

### Characterization of complex

### Example 12

¹H-NMR spectra are obtained with Varian NMR, 300 MHz, solvent D₂O, external reference TMSPO=0 (Figures 1-8).

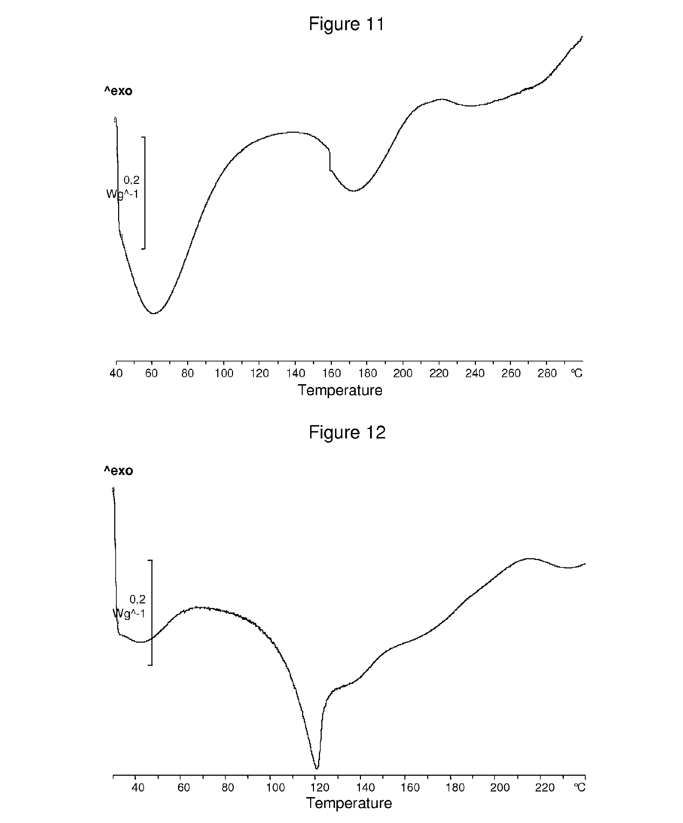

Thermograms are obtained with:
a) TA Instruments differential scanning calorimeter. The sample is heated at 5°C/min in the temperature range from 30°C to 300°C (Figures 8-10),
b) Mettler Toledo DSC822^{e} differential scanning calorimeter. The sample (4-6 mg) is placed in an unsealed aluminium pan with one hole and heated at 5°C/min in the temperature range from 40°C to 300°C (Figures 11-13).

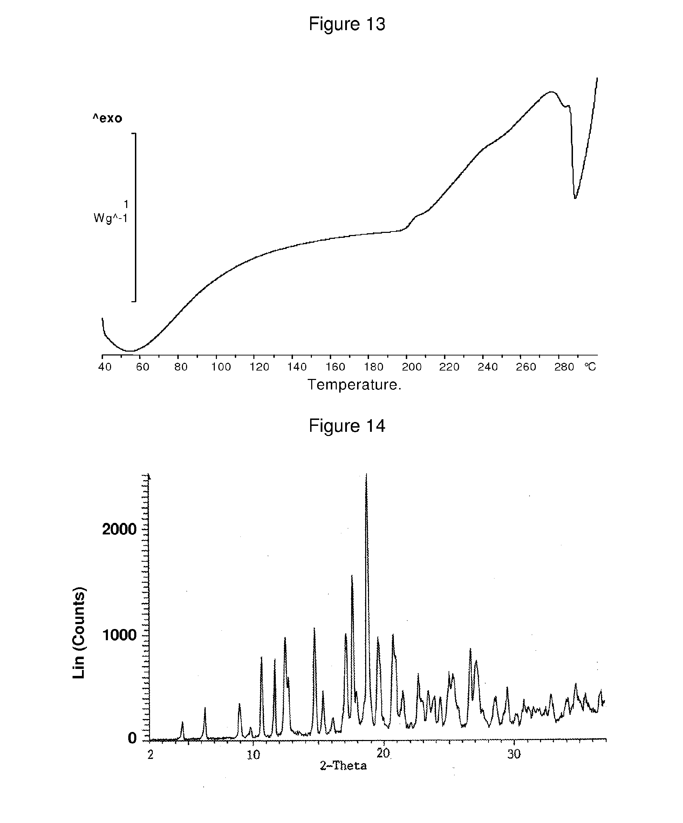

The powder X-ray diffraction diagram (Figures 14-19) by Siemens D 5000 diffractometer uses the reflection technique under the following conditions: CuK_{α} radiation, range between 2° and 37° (2θ), step 0.04° (2θ), integration time: 1 second.

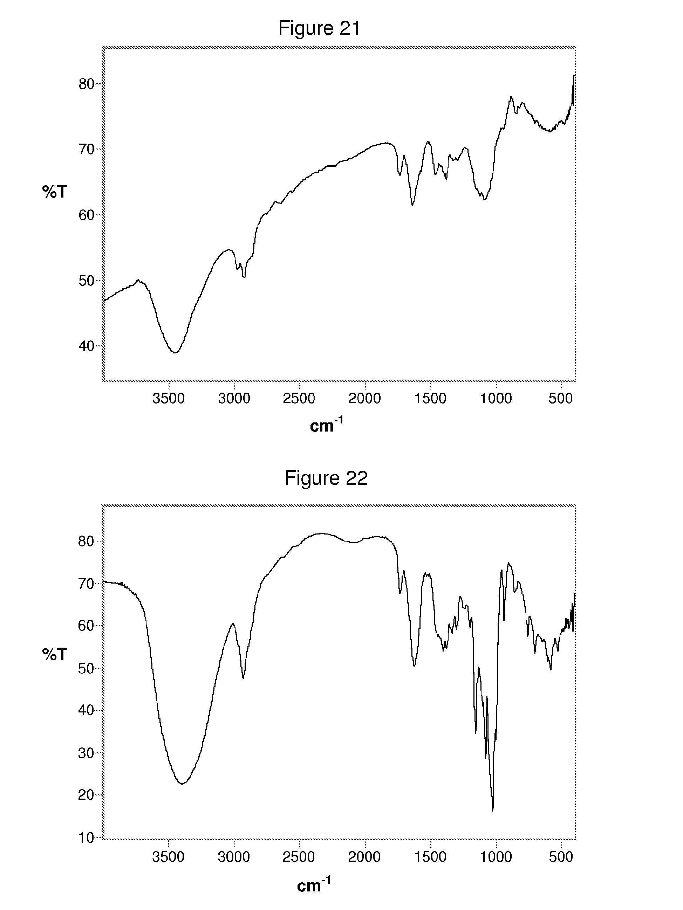

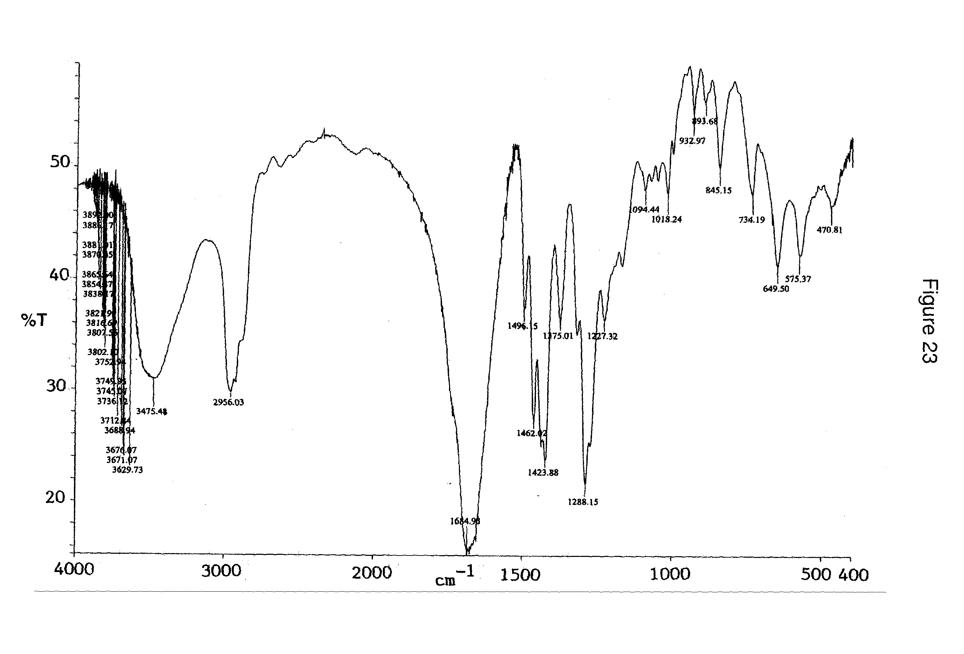

Infrared spectra are obtained with:
a) Bio-Rad FTS-60 spectrophotometer. Samples are analyzed in KBr (Figure 23).
b) Nicolet Nexus FTIR spectrophotometer. Samples are analyzed in KBr and scanned from 400 to 4000 cm⁻¹ with 16 scans and 2 cm⁻¹ resolution (Figures 20-22).

### Pharmaceutical tablet composition

### Example 13

Composition for the preparation of 1000 tablets comprising 4 mg of perindopril erbumine as active ingredient in one tablet:

| | |
|---|---|
| inclusion complex of example 2 (contains 20 % of perindopril erbumine) | 20 g |
| hydroxypropylcellulose | 2 g |
| wheat starch | 5 g |
| lactose | 85 g |
| magnesium stearate | 3 g |
| talc | 3 g |

## Claims

1. Amorphous perindopril erbumine.

2. A pharmaceutical composition comprising a therapeutically effective amount of amorphous perindopril erbumine with one or more pharmaceutically acceptable carriers or excipients.

3. A pharmaceutical composition according to claim 2 wherein said pharmaceutical composition comprises one or more additional pharmaceutically active component.

4. A pharmaceutical composition according to claim 3 wherein the additional pharmaceutically active component is a diuretic.

5. A pharmaceutical composition according to claim 4 wherein the diuretic is indapamide.

6. Use of amorphous perindopril erbumine for the preparation of a pharmaceutical composition for use in the treatment of cardiovascular diseases.

7. Use according to claim 6 wherein a cardiovascular disease is selected from the group consisting of hypertension and heart failure.
